(19) Europäisches Patentamt
European Patent Office
Office européen des brevets

(11) **EP 4 180 088 A1**

(12) **EUROPEAN PATENT APPLICATION**

(43) Date of publication:
**17.05.2023 Bulletin 2023/20**

(21) Application number: **21208387.7**

(22) Date of filing: **16.11.2021**

(51) International Patent Classification (IPC):
**A61N 5/10** (2006.01)    **G06N 3/08** (2023.01)
**G06N 20/00** (2019.01)    **G16H 20/30** (2018.01)
**G16H 20/40** (2018.01)    **G06N 5/00** (2023.01)

(52) Cooperative Patent Classification (CPC):
**A61N 5/103; G06N 3/08; G06N 5/00; G06N 20/00;
G16H 20/40; G16H 50/20;** A61N 2005/1041

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **HEESE, Harald Sepp
Eindhoven (NL)**

• **VIK, Torbjoern
Eindhoven (NL)**
• **ISOLA, Alfonso Agatin
Eindhoven (NL)**
• **BONDAR, Maria Luiza
Eindhoven (NL)**
• **BROSCH, Tom
Eindhoven (NL)**
• **BAL, Matthieu Frédéric
Eindhoven (NL)**

(74) Representative: **Philips Intellectual Property &
Standards
High Tech Campus 52
5656 AG Eindhoven (NL)**

(54) **A MACHINE LEARNING BASED SYSTEM FOR GENERATING OBJECTIVE FUNCTIONS FOR RADIATION THERAPY TREATMENT PLANNING**

(57) System (OGS) and related method for generating an objective function for use in radiation treatment, RT, planning. The system may include a machine learning model (M) and a training system (TS) for training the model (M) based on training data. The training data may include previous (at least partial) RT plans, and a user awarded ranking thereof. The model, once trained, may be used as the objective function. The system allows a user to turn, in a defined manner, clinical objectives or goals into a computable objective function which can be used for RT planning.

FIG. 2

EP 4 180 088 A1

**Description**

FIELD OF THE INVENTION

The invention relates to system for generating an objective function for use in radiation therapy planning, a system of radiation therapy planning, a training system for training a machine learning model for use in any one of such systems, to related methods, to a computer program element, and to a computer readable medium.

BACKGROUND OF THE INVENTION

Cancer is a leading cause of death in industrialized nations around the world. Well over half of cancer patients receive treatment by Radiotherapy ("RT"). In RT, high energy ionizing radiation is applied to the patient, either from outside the patient (external RT) or from within (brachytherapy). The goal is to destroy cancerous tissue whilst preserving non-cancerous, healthy tissue. In some types of external RT, X-radiation in the megavolt (MV) range is used. The total radiation dose is administered in portions (also known as "fractions") over time as per a treatment plan, such as once per day over the course of 2-4 weeks for example.

The preparation of the treatment plan is an important but time-consuming process. The user of a Treatment Planning System often strives to come to favorable compromise between tumor irradiation and sparing of normal tissue.

Treatment planning systems support this task by providing low-level objectives as well as high-level treatment goals to the user, which can then be combined and parameterized to express the individualized treatment goals for the patient at hand, and to achieve an optimal treatment plan meeting the expressed goals.

Based on these objectives and goals, treatment planning systems rely on an optimization engine that tunes the delivery parameters of a radiation therapy treatment plan such that the respective goals are achieved to the best possible extent as measured by objective functions. This process is often called inverse optimization.

The underlying mathematical problem formulation for inverse optimization in RT suffers from ill-posedness. While some treatment goals and plan characteristics can be well-described and well-managed in the above framework, there are also desirable dosimetric characteristics or treatment goals, for which the typical user is not capable of directly translating them into mathematical objective functions for use by the planning system.

SUMMARY OF THE INVENTION

There may therefore be a need for systems or methods to facilitate treatment planning.

An object of the present invention is achieved by the subject matter of the independent claims where further embodiments are incorporated in the dependent claims. It should be noted that the following described first aspect of the invention equally applies to the system of radiation therapy planning training, to the system for training a machine learning model for use in any one of such systems, to the related methods, to the computer program element, and to the computer readable medium.

According to a first aspect of the invention there is provided a computing system configured for generating an objective function for radiation treatment, RT, planning.

In embodiments, the system is based on a machine learning model, and the system comprises a training system configured to train the machine learning model based on training data.

The system allows a user to turn, in a defined canonical manner, clinical objective or goal a clinical goal into a computable objective function.

In embodiments, the system includes a model analyzer configured to analyze the model, once trained by the training system, to generate a representation of the objective function. The model analyzer is optional however as in a preferred embodiment, it is the trained model itself that is or includes the objective to be generated. In other words, the objective function is generated by learning from data and adapting (parameters) of the machine learning ("ML") model based on the training data. Thus, it is the so trained model itself that may be used as an objective function. In this manner, a precise computable function is obtained for the initial, merely intuitively accessible, clinical goal the user had in mind. Thus, the proposed setup helps the user to make the intuitive, implicit clinical goal or objective explicit in form of the trained model that can be used in objective-function based RT plan optimization algorithms. The model may be used as a cost or utility function, depending on the exact optimization setup.

In embodiments, the training data includes i) plurality of previous radiation treatment plans and ii) a user defined ranking of the plurality of previous radiation treatment plans. The treatment plans may be partial (it is not necessary herein to use complete plans) and includes in particular respective dose (distribution) maps.

In embodiments, the system comprises a user input interface, configured to allow a user for providing the said training data.

In embodiments, the user inface includes a graphical user interface.

In embodiments, the, or a (another), user interface is configured to allow the user to store the representation of the objective function in a data storage, preferably in association with a user selectable identifier that identifies an objective, criterion or goal. The system thus allows user customization.

In embodiments the, or a, user interface is configured to allow the user to select the objective criterion, wherein, upon such selection, the objective function is included as an objective into an RT planner system, the RT planner system to run an optimizing procedure driven at least by the objective function to compute a new treatment plan.

In embodiments, the optimizing procedure is of the inverse planning type. In embodiments, the machine learning model is a regression type model or is of the generative type.

In embodiments, the model is of the artificial neural network type.

In another aspect there is provided a system for computer-assisted radiation treatment planning, comprising:

input interface for receiving a selection of an objective function generated by a system of any one of the embodiments mentioned above; and

an RT planning module configured to run an optimizing procedure driven at least by the selected objective function to compute a new treatment plan.

In yet another aspect there is provided an RT arrangement that comprises the system of any one of the above embodiments, and a radiation delivery apparatus controllable by the new treatment plan.

In a further aspect there is provided a computer implemented method for supporting RT planning, comprising generating an objective function for RT planning.

The method further comprising providing the objective function for use in an RT planning algorithm. The algorithm may be of the iterative type. (Intermediate) dose maps and fluence maps/control parameters are computed in the iterations so as to improve the objective function, until a stopping condition is fulfilled.

In a further aspect still there is provided a computer program element, which, when being executed by at least one processing unit, is adapted to cause the at least one processing unit to perform the method.

In a further aspect there is provided at least one computer readable medium having stored thereon any one of more of i) the program element, ii) the (trained) machine learning model, and iii) the representation of the objective function. In preferred embodiments, the trained machine learning model is the objective function.

What is proposed herein is an ML-based approach for generating customizable objective functions based on user-defined criteria such as the ranking of prior plans/dose distributions. The ranked plans represent training data. The objective function is then learned from this user input by using a machine learning training algorithm. The proposed system builds up a machine learning model that is capable of outputting ranking scores as a quality metric. The model is preferably configured to represent a differentiable function that is non-negative. The model is either used as is or is used as a basis to derive therefrom another differentiable real-valued non-negative function. The model or the function to derived may be made available as a user-selectable objective function for an optimization procedure to compute a treatment plan, such as inverse planning algorithm.

The proposed system and method address the asymmetry in knowledge that exists in that user may only know intuitively which of existing plans is better than another, but they may not know how to formulate an objective function explicitly for use in a planning algorithm. For example, the medical user may know how to assess a treatment plan such as by reviewing plan qualities/metrics, such as low dose bath behavior, dose leakage, movement patterns of collimator leaves, fluence utilization, and so forth. Similarly, some plans may include artefacts which the user is able to notice but is unable to characterize or efficiently work against using an existing set of objectives and treatment goals.

Such plan qualities may be expressed as a matter of principle as functions (objective function) of the dose map, but the user may not know how to formulate such functions in mathematical terms to yield a non-negative differentiable functional expression that can return a concrete quality metric when presented with a given plan. The proposed system harnesses the fact that medical users are more likely to be able to merely compare plans against a given, merely intuitive objective/goal/criterion, and decide which of the two plans is the "better" one. The proposed system is configured to turn this implicit knowledge into a concrete computable expression, that is the trained machine learning, and use this as an objective function for an RT planning algorithm.

"*user*" relates to a person, such as medical personnel or other, operating the treatment delivery apparatus or overseeing of or taking part the RT treatment, including planning. In other words, the user is in general not the patient to be treated.

In general, the term "*machine learning*" includes a computerized arrangement (or module) that implements a machine learning ("ML") algorithm. Some such ML algorithms operate to adjust a machine learning model that is configured to perform ("learn") a task. Other ML operate direct on training data, not necessarily using such as model. This adjusting or updating of training data corpus is called "training". In general task performance by the ML module may improve measurably, with training experience. Training experience may include suitable training data and exposure of the model to such data. Task performance may improve the better the data represents the task to be learned. "*Training*

*experience helps improve performance if the training data well represents a distribution of examples over which the final system performance is measured*". The performance may be measured by objective tests based on output produced by the module in response to feeding the module with test data. The performance may be defined in terms of a certain error rate to be achieved for the given test data. See for example, T. M. Mitchell, "Machine Learning", page 2, section 1.1, page 6 1.2.1, McGraw-Hill, 1997.

BRIEF DESCRIPTION OF THE DRAWINGS

Exemplary embodiments of the invention will now be described with reference to the following drawings, which, unless stated otherwise, are not to scale, wherein:

Fig. 1 shows a schematic block diagram of a radiation therapy arrangement;
Fig. 2 shows a schematic block diagram of a computer implemented system for supporting radiation treatment planning;
Fig. 3 shows a machine learning model which may be used in some embodiments in the system of Fig. 2;
Fig. 4 shows a training system that may be used to train a machine learning model;
Fig. 5 shows a flow chart of a method of facilitating radiation treatment planning;
Fig. 6 shows a flow chart of a method of using objective function(s) in radiation treatment planning; and
Fig. 7 shows a method of training a machine learning model.

DETAILED DESCRIPTION OF EMBODIMENTS

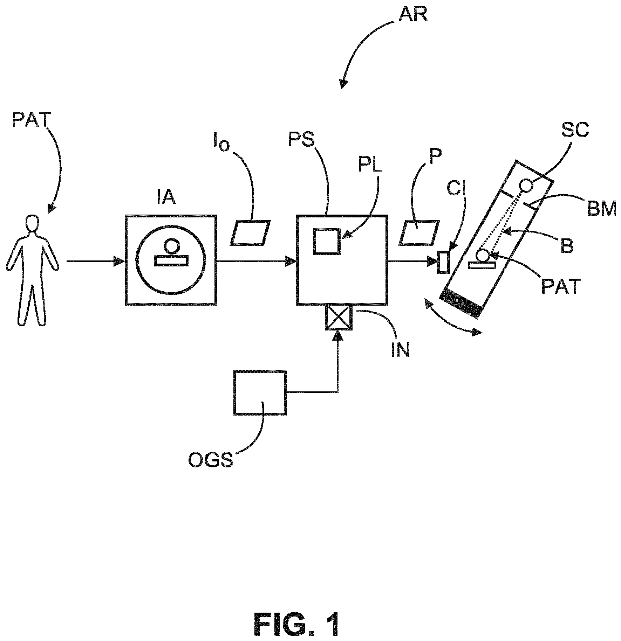

With reference to Fig. 1, there is shown a schematic block diagram of a computerized radiation therapy ("RT")-arrangement AR. Broadly, the RT arrangement AR may include a treatment device TD configured for RT delivery based on a treatment plan P. The arrangement AR may further include a treatment planning system PS configured to compute the said plan. Very broadly, as proposed herein, the arrangement includes a system OGS configured to assist a user to operate the planning system PS. Based on user input, the system generates objective function(s) $f$ for use in planning system PS. Before explaining operation of the new arrangement in more detail, some context around RT is provided first.

External radiation therapy is mainly envisaged herein although the principles described herein may also be applied to internal radiation therapy. In external radiation therapy, high energy radiation is delivered to a patient to kill off cancerous tissue. The objective is to kill off as much of the cancerous tissue as possible, whilst sparing non-cancerous tissue. The cancer killing radiation is delivered by the treatment device TD such as a linear accelerator ("linac"). In linacs, a beam B of high energy x-ray photons, such as in the megavolt range are generated by a source SC. During treatment, The patient is exposed in a pre-mediated manner to the beam and thus the radiation delivered by the treatment device TD.

Types of X-ray based external RT envisaged herein include intensity-modulated radiation therapy ("IMRT"), volumetric-modulated arc therapy and others. Specifically, in X-ray based external RT, the radiation is delivered at certain doses from a multitude of different spatial directions. More specifically, radiation treatment beams propagating along the said different spatial directions can be made to intersect preferably at the lesion (the target volume, "PTV") to be treated, thus delivering a higher dose there, whilst leaving a lower dose at surrounding non-cancerous tissue, in particular organs at risk ("OAR"). The applicable doses to be delivered and the spatial directions relative to the lesion site through which the dosages are to be delivered are defined in the treatment plan.

The treatment plan is computed by a planning module PL of the treatment planning system PS. The planning module PL may be implemented by a computing system.

Computing the treatment plan is in general a complex mathematical operation that may consume time and computational resources, and hence electrical energy. Preferably, high performance computing processors such as those of multi-core design may be used to secure a reasonable return time.

Computing the treatment plan ("the planning problem"), may be formulated as an optimization the problem. Specifically, one or more objective functions are formulated subject to one or more constraints. Generally, the objective function and the constraints define the manner in which the treatment dose is to be delivered given location, shape and extent of the cancerous lesion. More specifically yet, the objective function(s) and the constraint(s) describe how much dosage is to be delivered in which regions. A clinician prescribes for instance the average dose in a certain area, or the maximum or minimum dose to be delivered and formulates these objectives and constraints.

Planning imagery is previously acquired by a medical image modality IA, such as a computed tomography scanner CT, a magnetic resonance imager (MRI), or a nuclear imaging modality such as PET/SPECT. Based on this planning imagery $I^0$, the cancerous target region/volume PTV may be identified. This initial planning imagery $I^0$ is preferably 3D so as to capture the 3D shape and extent of the cancer lesion situated usually at least partly, if not fully, inside the patient. The target volume TV is identified either manually by delineation in the imagery, or the delineation is done automatically or semi-automatically by a segmentation algorithm. The identified tissue includes the volume PTV to be treated where the cancerous tissue resides plus a certain safety margin. Volumes of non-cancerous tissue, such as the above-mentioned OARs, may also be segmented or otherwise identified in the planning imagery. OARs are to be spared from radiation exposure, or deposited radiation is at least not to exceed certain maximum thresholds as formulated in the planning constraints.

The planning module PL implements the said planning algorithm to find a solution to the planning problem. In particular, inverse planning algorithms may be iterative and compute how much dosage is to be delivered from which spatial position over a certain period of time. In the iterations, the objective function is improved until an acceptable treatment plan is found. The total dose is deliverable in fractions. In other words, parts of the dose are delivered over a treatment course of days or weeks, in multiple treatment sessions. A treatment session for a fraction is usually once per day. In each such treatment, a certain dose part of the total dose envisaged is delivered from the computed spatial positions.

The computed RT treatment plan P may include in particular a quantity known as a "fluence map". The fluence map $\varphi$ defines the intensity of the treatment beam across its cross section.

Based on the computed fluence map, a machine control program is computed by the planning module PL to control operation of the treatment delivery device TD (such as the linac or other). For example, in IMRT, a leaf sequencing tool computes the control program to control a beam modulator BM of the delivery device TD. For example, the beam modulator may include a multi-leaf collimator. The control program may control position and timing motions of leaves of the MLF collimator. For instance, in modulated radiation therapy, the treatment device TD includes a treatment head with the beam modulator BM such as the said multi-leaf collimator that can realize different beam shapes. The cross section of the beam B may be divided into beamlets where different dosages can be realized in a spatially resolved manner. The computed control program of the treatment plan may prescribe different collimator settings that may vary as the treatment head is moved (by a rotatable gantry) to different spatial positions around the target volume. The collimator settings may prescribe how leaf elements of the multi-leaf collimator are to be moved to realize the different beam shapes. The control parameters may be passed via a control interface CI to the delivery apparatus TD to effect the treatment.

Preferably prior to actual plan delivery/treatment, as a quality check for example, simulation tool of the planning module PL may process the control program to compute deliverable dose distribution $d$, if the control program (that is, the current plan) were to be used. The computed dose distribution d may be stored as a spatially resolved file or data structure, also referred to herein as the dose map $d$. The deliverable dose distribution map $d$ may be optionally superimposable on the planning image to represent the dose per location. The dose maps d envisaged herein are preferably 3D, although formulations in 2D are not excluded herein.

In order to facilitate (radiation) treatment planning, the planning system PS incorporates, or interacts with, a planning facilitator system OGS ("facilitator"). The facilitator OGS is preferably user interactive, but automatic embodiments are also envisaged. The facilitator OGS is operable to generate input data for processing by the planning module PL. Input data generated by the facilitator OGS may be passed on through a suitable interface IN to the planning module PL. The planning module PL implements the above-described optimization procedure to compute the treatment plan $P$. The planning module PL may use the RT planning algorithm to compute the plan. As said, such algorithms may be based on a (numerical) optimization procedure. The procedure may be driven by one or more objective functions. The one or more objective functions may represent clinical objectives or goals in relation to the RT treatment plan P of the given patient PAT. Thus, (clinical) objectives may be formulated as objective functions. Specifically, the plan is computed by planner PL whilst improving objective functions. The proposed facilitator OGS facilitates operation of the planning module, or specifically the optimization algorithm, by assisting the user to formulate the said one or more objective functions. Thus, the facilitator OGS may be understood as a computer assisted objective function generator. As mentioned, operation of the facilitator OGS in generating the objective functions is user-interactive via one or more user interfaces ("UI"). The UI may include a graphical user interface(s) ("GUI"). In the following, we will be referring herein for simplicity to "the" objective function $f$, with the understanding that there may be more than one such functions generated, as a system of objective functions $f = f^i$. For example, a plurality of such functions may be generated by the facilitator OGS for a single (clinical) objective, or one or more functions for any given objective out of a plurality of objectives. Indeed, more often than not, plural such a system of (plural) objective functions may be required for an adequate formulation of the planning problem which the optimization procedure (planning algorithm) is configured to solve.

Conceptionally and in more detail, the optimization procedure may be described as follows: the planning imagery $I^0$, such as a computed tomography (CT) scan may define a grid of voxels. Dose per voxel is a primary quantity of interest. This dose per voxel relationship may be defined as a dose map. The dose map may be represented as a matrix. For efficiency, the dose map matrix may be "unfolded" into a dose vector $d$, with length equal the total number of patient voxels for the region of interest.

The control variables $\kappa$ of interest for controlling operation of the treatment device TD are the beamlet intensities or fluences. Beamlets can be conceptualized as a radiation field of the beam B per projection direction divided up into a regular grid with elements "bixels", which may be coded spatially into matrix or, for computational efficiency, as a

vector $_\chi$. A mapping from beamlet intensities to voxel doses may be represented by dose-influence matrix D. A basic radiation therapy optimization problem may then be formulated as:

$$arg\ min_d\ f(d) \qquad (1a)$$

$$D\chi = d \qquad (1b)$$

$$\chi \ge 0, \qquad (1c)$$

Eqs (1b-c) are constraints, where eq (1b) require the deliverable dose to equal the planned dose whilst eq (1c) is a sanity "checker" to exclude bogus solutions with negative fluence which of course do not exist. Eq (1a) represents an objective function $f$ that controls the planning optimization to find the best dose map and hence the associated control parameters $_\chi$ or fluence maps. Generally, the objective function $f$ measures aspects of the dose map such as the manner in which it varies relative to PTV and/or OARs, its local or global shape, etc. As noted above, a system $f_i$ of such objective functions may be used at (1a) instead of a single objective function f. The notation "$f$" is used herein for the objective function in the context of the planning optimization setup eq (1a-c), whether a single such objective function or a system of such objective functions is used. A plan, as computed by procedure (1a-c) as understood herein is to include in particular a dose map $d$. See D Craft "Multi-criteria optimization methods in radiation therapy planning", available online at arXiv:1305.1546v1, May, 2018.

However, it has been found that specifically describing in dose domain the dose objectives individually, per pixel or region or even globally, is cumbersome and not always easy for the user to do. The precise mathematical form of the objective function $f$ may be difficult to guess based on the clinical objective/goal one has in mind. Thus, it may be counter-intuitive for the clinical user to formulate mathematical objective function(s) that adequately reflect the clinical objective which user has in mind. The facilitator system OGS thus assists users in "translating" clinical objectives, including modifications informed by their personal preferences, into computable objective function(s) for use in treatment planning. Once the mathematical form and configuration of the objective function is established, the facilitator system OGS, upon user input, may pass the objective function $f$ on to the planning module PL. Thus, as noted briefly above, the planning facilitator OGS may be understood as an objective function generator system. The planning facilitator OGS may be fully or partly integrated into the planning system PS or, more specifically, into the planning module. However, this is not necessarily required herein, and the facilitator system OGS may be arranged instead as a stand-alone component. The generated objective function $f$ for planning may be passed on through a suitable communication interface to the planning system PS and/or planning module PL. The generated objective function $f$ may be stored on an external or internal memory such that the planning module PL can access the function $f$. The stored objective function $f$ may be loaded by the planning module PL / planning system PS as required.

Broadly, the planning facilitator OGS allows the user to translate intuitive medical radiation treatment objectives into (effectively) computable (in the Turing sense) mathematical functions or computational routines on which the planning module can act on. The generated objective function may control the optimization procedure. The generated objective function $f$ may be represented as circuitry and/or data structure, as routine, in software, in software and hardware, as a signal, etc., for use in the RT optimization algorithm, such as in an inverse planning algorithm.

Conceptually, the facilitator OGS implements a mapping from possibly intuitive and only vaguely defined clinical objectives or goals into the space of mathematical objective functions. The clinical goals may differ with clinical user as they may depend on experience. Such clinical goals may relate to any one or more of:

the extent to which the target is inside a (high) isodose line (e.g., TV is inside the 95% isodose line),
geometric/alignment conformality, that is the extent to which a shape of a high dose isodose lines conforms a shape of the target TV,
shape of the dose profile, such as the steepness of the dose-gradient in a region of interest/OAR adjacent to the target TV,
extent to which the target TV or an organ-at-risk OAR is exposed to a dose higher than a user-prescribed maximum value,
unevenness of the dose distribution surface such as presence of hot and cold spots, etc, in particular by taking their location relative to TV and/or OAR into account. For example, a cold spot in the target TV close to the spinal cord might be acceptable, and so may be a hot spot in the middle of the target TV, but a hot spot inside an OAR might not be acceptable and attract a low score in relation to this metric,
shape and/or the position of the maximum dose point(s) in the plan/dose distribution,

shape of low isodose lines, as their shape can reveal unexpected dose distributions.

Whilst there may be agreement on certain general oncological principles and common approaches among practicing clinical users, when it comes to planning RT treatment for a given patient, individual preferences and deviations may still remain. Such preferences may be the result of certain individual, specific practical experiences that may differ from clinical user to user. And it is in particular such highly individualized user preference in RT planning that made it previously difficult, if not impossible, to formulate the above mapping in analytical standardized form from the outset. It is therefore proposed herein for the facilitator OGS to use machine learning to address this need. The facilitator OGS in configured to harness at least two aspects: i) the medical experience as embodied by an existing corpus of previously computed treatment plans, and ii) the individual user's treatment preference. The user can thus customize the planning by ranking the example plans drawn from the existing corpus and the machine learning model is then trained to learn this "ranking function" as prescribed by the user. A machine learning model $M$ can be adapted in a training or learning phase based on the user ranked treatment plans. The user may have different clinical goals in mind, and the facilitator can assist on learning a corresponding plurality of differently trained models, one or more such models for each goal $i$. Once learned, the one (or more) machine learning model $M$, $M_i$ are configured to compute a (respective) scalar value (a number) that represents the user's ranking in respect to a given goal. The so trained model(s) can then be used as an objective function in the planning optimization procedure by the planning module PL. That is, in embodiments, it is the so trained machine learning model(s) itself that is used as the objective function. The model is preferably configured as a model that is differentiable with respect to its parameters that are adapted during the learning. It is ensured by the mode architecture and/or learning, that model is non-negative function. The ranking score as producible by the model $M$, $M_i$ may be used to control the planning optimization procedure. More details on the learning procedure will be described further below.

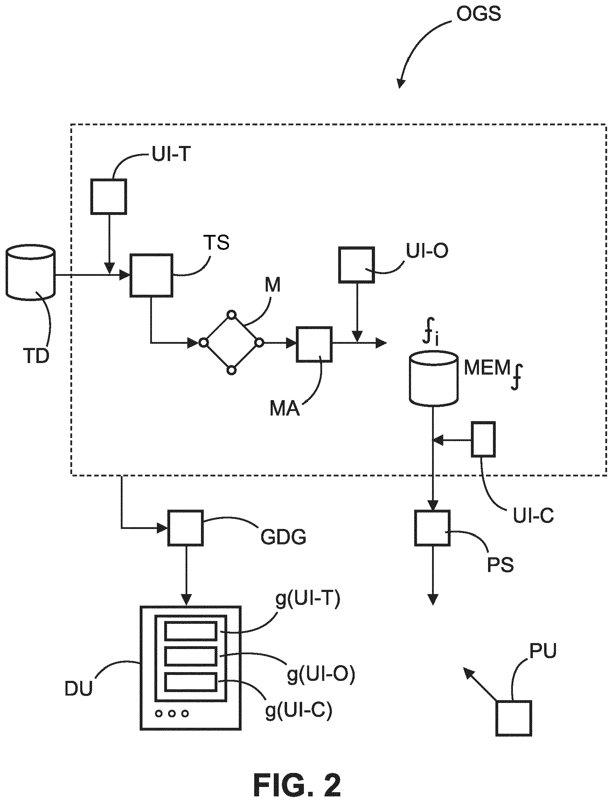

In more detail, reference is now made to the block diagram in Fig. 2 which represents components of the planning facilitator system OGS. The facilitator system OGS may support one more UI functionalities. One UI functionality, the input UI functionality UI-T, is configured to allow the user to furnish the facilitator OGS with ranked plans as input data for processing. An ML training system TS uses the ranked plans as training data to train a machine learning model. The ML training system TS may be integrated in the facilitator OGS as shown, or may be an external resource to which the training task may be outsourced by the facilitator OGS. The trained machine learning model M may form the objective function that represents the clinical objective/goal as derived from the ranking of plans received as input. The so computed objective function $f$ may be stored upon request in a memory $MEM_f$. Objective function memory $MEM_f$ may be internal or external to the facilitator system OGS. In the latter case, a central objective function repository may be envisaged where clinical users can store/upload their objective functions. The request for storage and optionally other functions may be facilitated via output UI functionality UI-O. The objective function $f$ may be supplied as input to the planning module PL via selector UI functionality UI-S. All of the three UI functionalities UI-T, UI-O, UI-S are optional, as (fully) automatic embodiments of the facilitator system OGS are also envisaged. In particular, output and selector UI functionalities are optional, in particular in partly-automatic embodiments.

Any one or any two, or indeed all three, user interface functionalities UI-T, UI-O, UI-S may be supported as graphical user interfaces GUI to yet better facilitate operation of the system and interaction with the user. The system is preferably not fully automatic (although this is not excluded herein in embodiments), but relies on user input and optionally on interaction with the user in one more iterations. A graphics display generator GDG is included or is in communication with the facilitator system OGS. The graphics display generator GDG generates graphical user interface representations g(GUI-T), g(GUI-O) and/or g(GUI-S) which may be displayed by and on a display unit DU. The graphics display generator GDG may be coupled to an event handler logic, operable to intercept user interaction and to cause in response thereto, the respective function to be executed. Any two or all three UI functionalities may be supported as separate GUIs or may be provided in a single GUI, as required.

The input data UI UI-T may be arranged as training data explorer tool. Input data UI UI-T is configured to allow user searching for prior plans in memory (such as in a database or other data repository) based on for example any one of patient type, ROI, target PTV, OARs etc. and combinations thereof. This memory holding the previous plans may be referred to herein as a training data storage TD. The input data user interface UI-T allows the user to select training data from plans as held in the training data storage TD. The training data storage TD may be a medical database that stores previous plans of previous patients for example. The input data user interface UI-T allows the user to operate suitable retrieval logic and storage interface software to interact with the training data storage TD to effect retrieval of such previous treatment plans. In particular, the respective dose maps $d$ may be so retrieved and may be visualized. It is not required herein to use full previous plans, but mere parts of the plans may suffice. A plan for present purposes includes the deliverable/simulated dose map $d$. The plan may further include contextual data, such as segmentations for any one or all of PTVs and/or OARs. Prescribed minimal ("*min*") and maximal ("*max*") dose levels for PTV and OARs, respectively, may also be included in the retrieved plan, and so are quantifications of priorities of the *min*/*max* dose levels.

A browsing function may be supported by the input data UI UI-T. The browsing function may allow the user finding plans whose characteristic corresponds to the clinical objective the user has in mind. The facilitator system OGS relieves the user from the burden of formulating precise data characteristics to find the plans that may correspond to the clinical goal the user has in mind. A mere visual review, a "scrolling" through, visualizations of previous plans rendered on the display unit by a visualizer component of the graphics display generator GDG, is sufficient for present purposes.

The input user interface UI-T functionality is configured to allow the user to rank according to their specific preference, the reviewed plans in respect of the current clinal goal/objective. The plans are ranked according to how well they represent the current clinal goal/objective of interest. For example, based on the associated dose maps, the plans are arranged in an order. Each dose map may be given a rank score, a number that is. The rank score may be explicitly awarded by selecting a score, or the ranking scores are automatically awarded based on a current order in which the reviewed plans are listed for example. The awarding of scores, the ordering in a list etc., may be supported by pointer tool operations (such as mouse-click or touch screen finger action, etc). Contextual data such as planning imagery for the respective plans may be associated as contextual data c with the respective dose maps to form the plan as a respective "packet" of training data. The input user interface UI-T thus allows the user to formulate instances $i$ of training data packets which may be formally described as $(x, y)^i$ or $[(x, y), c]^i$, where for each instance $i$, $x$ is the identified plan which may include in particular the respective dose map for this plan, $y$ is the ranking awarded by the user, and $c$ is optional contextual data associated with plan $x$. The input user interface UI-T is configured to allow the user to retrieve a plan from previous plans, and tag same with rank score $y$. In the following, for simplicity at times reference is made generically to the training data (pair) $(x, y)$, or $(x, y)^i$, it being understood that such reference may include the case where contextual data c is used $[(x, y), c]^i$. Instead of or in addition to the dose map $d$ corresponding to the plan, training input $x$ may further include plan delivery parameters of the current treatment plan such as beam positions, collimator shapes, beam intensity, monitor units, etc., or the fluence maps $X$. The contextual data c may include any one or more of: the planning image $I^0$ for the current treatment plan, segmented/delineated ROIs for target structure PTV and risk organs OARs, or high-level treatment goal description (such as listed above at a)-g) ) associated with this treatment plan, if available.

In embodiments, the user interface UI-T is configured to allow the user to rank the training set on plural goal/objectives simultaneously, ie, to independently compare the training samples with respect to different such goals/objectives.

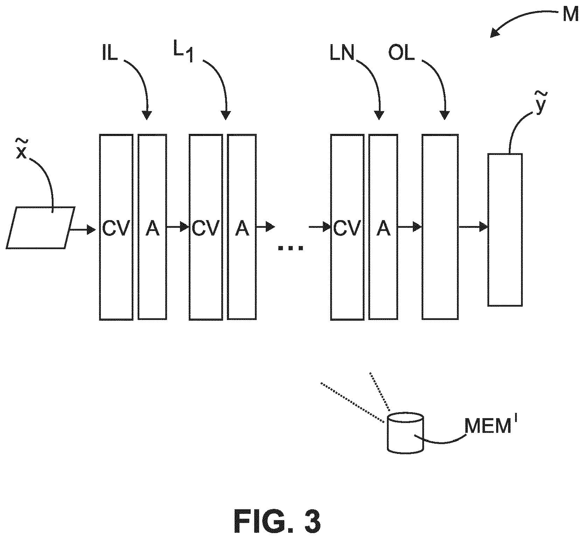

The tagged input data $(x, y)^i$ is then passed on to the machine learning training system TS configured to train ML model M based on the training data $(x, y)^i$. The training/learning procedure can be a one-off, or can be done repeatedly as required. A neural network type model may be used as will be explained in more detail, but this is not a necessity herein. Existing algorithms such as of the back-propagation type or more generally gradient based can be used to adapt an initial set of parameters of the model based on the machine learning objective function $f_j$ that is the trained model $f_j=M_j$, in case multiple models are trained for different objectives $j$. Operation of the training system TS will be described in more detail below at Fig. 3. After training, once the model is sufficiently trained, the model may be stored in memory MEM$f$. The so trained model $M_j$ thus represents the objective function $f_j$.

The output data user interface UI-O may be used to effect storing the ML model M as objective function. In addition, or instead, the output data user interface UI-O may be configured to allow user annotating the trained model $M_j$ with an identifier, a string, such as a natural language expression or other apt symbology. The identifier is a reference to, or description of, the respective medical objective/goal the user had in mind when formulating the ranking of the training data $(x, y)$. This annotation facilitates later selection for passing on to the planning module PL as the objective functions composed herein may be used repeatedly in future planning sessions for the same or different patients. Thus, the above-described composing of the training data set by input data UI UI-T and annotation of trained model M by output data UI UI-O allows customization by user of the objective function generation as supported by the facilitator system OGS.

It will be understood from the above that the initial training set can be chosen and/or ranked according to different objectives separately so that different models $M_j$ emerge, each representing a different intuitive clinical objective/goal, the system of trained models $M_j$ thus forming the above-mentioned system of objective functions. Different training data instances may be used for different objectives. The one or more trained models $M_j$ may be stored as said in memory MEM$_f$, preferably annotated by operation of the output data user interface UI-O.

Planning preparation user interface UI-S allows the user at a later stage to designate a specific set of one or more stored objective functions (eg, the previously trained models $M_j$). The designated objective functions may then be made available to the planning module PL to control the optimization procedure to compute a new plan P for the given patient PAT.

Consistent with the above mentioned training procedure based on training data pairs $(x, y)^i$, the trained model M expects as input a plan (eg, a dose map), not necessarily from the training data, and produces as associated output a ranking score (a number that is) for the input plan. The ranking is consistent with the initial ranking as imposed by the user on the training data. Depending on how the ranking score was encoded, the magnitude of the ranking score corresponds to and varies with how well the plan corresponds to the intuitively formulated clinical objective/goal. For example, the higher the ranking score, the better the correspondence, with lower values indicating that the plan is of lesser relevance for the clinical objective/goal at hand. The ranking scores may be normalized values from a pre-defined interval, such as the unit interval from zero to 1 or any other pre-defined interval. Preferably, as most optimization procedures as explained above at eqs(1) are naturally formulated in terms of a cost function minimization, it may be preferred to encode the ranking scores in an "inverse" manner, so that a lower ranking score represents a better correspondence. The facilitator system OGS may include an encoder logic that remaps the user awarded scores in this inverse encoding, if required.

The one or more trained models $f_j = M_j = M$ may be used in deployment phase by the planning module PL in the optimization procedure. The objective functions are evaluated on intermediate plans P' as generated by the planning module PL during the iterative optimization. The intermediate plans P', in particular their intermediate dose maps associated with the plans, are fed into the respective model M to obtain a ranking value as a measurement. The optimization algorithm is so configured that, during its iterations, parameters of the plan are adapted so as to improve the ranking as computable by the model $f=M$. For example, in a cost function-based optimization, the parameters of the plan are adjusted so as to reduce the values returned by model $f=M$ over the iterations. Some optimization procedures in planning are gradient based, such as gradient-descent, conjugate gradients, Nelder-Mead, Newton, Raphson etc. Such optimization procedures may include an updater function that updates the plan parameters based on the gradient of the cost function. Operation of the optimization procedure may involve computing the gradient of the objective function $f=M$. The model architectures as envisaged herein are thus preferably differentiable to facilitate operation by the planning module PL of such gradient based optimization procedure. The computed differential or derivative of the objective function is used to drive or guide the iterative adaptation of the parameters of the intermediate plans P' to arrive at the final plan P.

Whilst it is envisaged herein to use the trained machine learning models M in and of themselves as objective functions, embedded in the treatment plan optimization, this may not necessarily be so herein in all embodiments. For example, the facilitator OGS may include an optional model analyzer MA to derive a simpler representation of the objective function from the trained model. This may be of interest if high throughput and conservative storage requirements are of interest. For example, the trained model may be fed by a series of plans from additional training data (not previously used in the training) to compute a range of ranking values. These rankings can then be stored in a look-up table ("LUT") for example. The LUT defines an approximation of the trained model M. To ensure this is differently, spline curves may be used. By this and similar other numerical methods, a simpler representation of the objective function may be obtained.

In one embodiment, the input data user interface UI-T affords the option for the user to specify a focus region (2- or 3-dimensional) in the dose map d or in the corresponding contextual data c or to apply a localized importance regularization in respect of the goal for which is currently ranked for. To this end, the user interface UI-T may include a delineator functionality for the user to delineate a 2- or 3-dimensional region which may be displayed by the graphics display generator GDG together with the dose map d and/or further contextual data c such as the planning imagery $I^0$ for the current training samples. The weighting is obtained by specifying the weight inside and outside the delineated region. Defining such localized regularization allows the user to fine-tune training operation. The training performance may be improved by providing "focus areas" for learning. An effect of the focus areas is that a learned dose map (and, optionally, the corresponding contextual data) restricted to the focus area should result in the same score, or, in other words, that the remaining information in the input data outside the focus region is irrelevant for the ranking.

Reference is now made to Fig. 3 which shows a machine learning model M as may be used herein in some embodiments. The machine learning model may be of the neural network type. However, as will be appreciated, the principles for objective function generation for facilitating RT planning as disclosed herein are not confined to neural network type models, and any other suitable machine learning model type or architecture may be used instead and are envisaged herein, such as statistical regression techniques, and others. ML models of the generative type are also envisaged herein, such as GANs ("Generative Adversarial Networks") or related or similar generative setups. GANs were described by Ian Goodfellow et al in "Generative Adversarial Networks", available at arXiv: 1406.2661, 2014. In GANS, the model M to be trained herein, is embedded in a model training network comprising at least two models, referred to as a generator (model) and a discriminator (model). The model M to be trained herein is arranged as the generator. A special cost function is used that incorporates conflicting objectives for the two models. In the GAN-learning algorithm, parameters of the models are adjusted so that an equilibrium state is reached, at which point the generator may be deemed trained. Model M is released from the framework as the trained model/objective function for use in RT planning as described above.

As said, whilst (artificial) neural network (NN) type models are not at the exclusion of other modeling approaches, it is in particular NN models that were found to yield good results. More particularly still, because the present approach envisages image type data, that is spatial data, as input such as the dose maps or planning imagery as contextual data, NN models of the convolutions type are preferred herein.

The machine learning model M may be stored in one (or more) computer memories MEM'. The trained model M may be deployed as a machine learning component that may be run on the computing device PU, such as a desktop computer, a workstation, a laptop, etc. or plural such devices in a distributed computing architecture. Preferably, to achieve good throughput, the computing device PU includes one or more processors (CPU) that support parallel computing, such as those of multi-core design. In one embodiment, GPU(s) (graphical processing units) are used.

The network M may comprise a plurality of computational nodes arranged in layers in a cascaded fashion, with data flow proceeding from left to right and thus from layer to layer. As mentioned, convolutional networks have been found to yield good result when processing image type or other spatial data as is the case for the dose maps or planning imagery for example that are processed by the model M.

In deployment, the input data $\tilde{x}$ is applied to input layer IL. The input data $\tilde{x}$ then propagates through a sequence of hidden layers $L_1$-$L_N$ (only two are shown, but there may be merely one or more than two), to then emerge at an output layer OL as a regression result $M(\tilde{x}) = \tilde{y}$, the ranking score. Thus, the regression output layer OL combines input that it receives from the previous layer $L_N$, and combines this input into the ranking score $\tilde{y}$. The output layer OL is preferably configured to yield non-negative scores, in consistency with constraint eq (1c) above. The tilde "~" notation indicates that the data is now new data, not from the training data set on which the model was trained. The input data $\tilde{x}$ in deployment are the intermediate plan data, in particular intermediate dose maps, as generated in the iterations of the planning optimization. And $\tilde{y}$ is the ranking score for controlling the update step in the planning optimization.

The model network M may be said to have a deep architecture because it has more than one hidden layer. In a feed-forward network mainly envisaged herein, the "depth" is the number of hidden layers between input layer IL and output layer OL. The number of hidden layers envisaged herein may well run into the 10s or even 100s, but a smaller number of hidden layers in single digits are not excluded herein.

Preferably, the hidden layers include a sequence of convolutional layers, represented herein as layers $L_1$ - $L_N$. The number of convolutional layers is at least one, such as 2-5, or any other number. The number may run into double-digit figures.

In embodiments, downstream of the sequence of convolutional layers there may be one or more fully connected layers. Alternatively, convolutional and fully connected layers may be interleaved. The output layer OL may be implanted as a full connected layer.

The hidden convolutional layers $L_m$ and optionally the input layer IL may implement one or more convolutional operators CV. Each layer $L_m$ may implement the same number of convolutional operators CV or the number may differ for some or all layers.

A convolutional operator CV implements a convolutional operation to be performed on its respective input. The convolutional operator may be conceptualized as a convolutional kernel. It may be implemented as a matrix including entries that form filter elements (weights) that form at least a part of the model parameters $\theta$ to be learned. It is in particular these weights that are adjusted in the learning phase.

The first layer IL processes, by way of its one or more convolutional operators, the input data of such input, such as the plan $\tilde{x}$, in particular the dose map (spatial dose distribution) as per this plan. Feature maps are the outputs of convolutional layers, one feature map for each convolutional operator in a layer. The feature map of an earlier layer is then input into the next layer to produce feature maps of a higher generation, and so forth with the last layer OL computing the desired ranking result $\tilde{y}$.

Convolutional filters CV in a given layer process an input feature map from an earlier layer into intermediate output, sometimes referred to as logits. An optional bias term may be applied by addition for example. An activation layer *A* processes in a non-linear manner the logits into the next generation feature map which is then output and passed as input to the next layer, and so forth. The activation layer *A* may be implemented as a rectified linear unit ("RELU"), although this is less preferred herein as the model M is then not differentiable (in the classical sense). Preferably and instead, the activation layer *A* is implemented as a sigmoid-function, *tanh*-function or any other suitable non-linear, but differentiable function. It is then the whole model $M(\tilde{x}) = \tilde{y}$ that is differentiable with respect to $\tilde{x}$, and M being the *N*-fold functional composition of the operators CV, A across the layers: $M = OL \circ (CV,A)_N \circ ... \circ (CV, A)_1 \circ IL\,()$.

For computational efficiency, especially when combined with processors capable of parallel processing, the input data $\tilde{x}$ in deployment and training, is represented as matrices. If contextual data is co-processed, $(\tilde{x},\tilde{c})$ or $(x,c)$, such as the dose map and planning imagery and optionally PTV, OAR segmentations and or patient medical record data, such data can be combined and presented as tensors for multi-channel processing by operators CV.

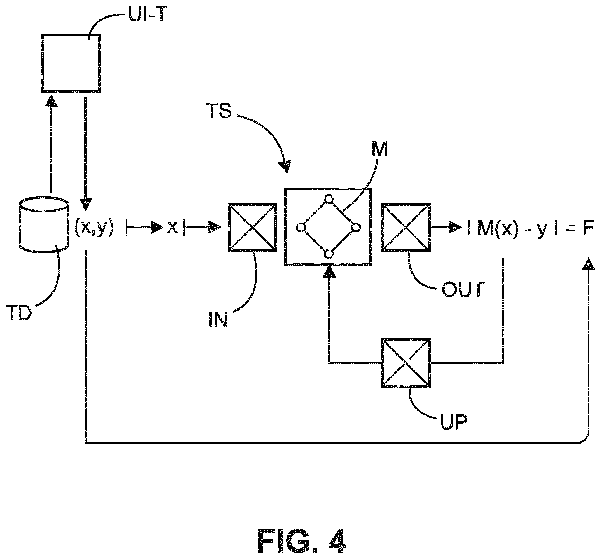

Reference is now made to Fig. 4 which shows a block diagram of the training system TS as incorporated in, or used in conjunction with, the above-described treatment planning facilitator OGS. In more detail, in the training phase, an architecture of the machine learning model M, such as the shown CNN network in Fig 3 is pre-populated with initial set of weights. The weights $\theta$ of the model NN represent a parameterization $M^\theta$, and it is the object of the training system TS to optimize and hence adapt the parameters $\theta$ based on the training data $(x_k, y_k)$ pairs. In other words, the learning can be formulated, similar to eq (1) above but unrelated thereto, as another optimization scheme where a cost function *F* is minimized although a "dual" formulation of maximizing a utility function may be used instead.

Assuming for now the paradigm of a cost function F, this measures the aggregated residue(s), that is, the error incurred between data estimated by the neural network model NN and the targets as per some or all of the training data pairs k:

$$argmin_\theta\, F = \sum_k || M^\theta(x_k) - y_k || \qquad\qquad (2)$$

In eq. (2) and below, function $M()$ denotes the result of the model M applied to training input x.

In training, the training input data $x_k$ of a training pair is propagated through the initialized network M. Specifically, the training input $x_k$ for a $k$-th pair is received at an input IL, passed through the model and is then output at output OL as output training data $M^\theta(x_k)$. A suitable similarity measure $\| \cdot \|$ is used to measure the difference, also referred to herein as residue, between the actual training output $M^\theta(x_k)$ produced by the model M, and the desired target $y_k$. For regression learning, least squares or more general $l_p$ measures may be used.

The output training data $M^\theta(x_k)$ is an estimate for target $y_k$ associated with the applied input training image data $x_k$. In general, there is an error between this output $M^\theta(x_k)$ and the associated target $y_k$ of the presently considered $k$-th pair. An optimization scheme such as backward/forward propagation or other gradient based methods may then be used to adapt the parameters $\theta$ of the model M so as to decrease the residue for the considered pair $(x_k, y_k)$ or for a subset of training pairs from the full training data set.

After one or more iterations in a first, inner, loop in which the parameters $\theta$ of the model are updated by updater UP for the currently considered pair $(x_k, y_k)$, the training system TS enters a second, an outer, loop where a next training data pair $(x_{k+1}, y_{k+1})$ is processed accordingly. Instead of processing pair by pair, a subset of pairs can be processed at once in batches and the outer loop proceeds from batch to batch until the current supply of training data instances is exhausted or until a predefined number of training data instances have been processed, etc. Batchwise processing is preferred.

The structure of updater UP depends on the optimization scheme used. For example, the inner loop as administered by updater UP may be implemented by one or more forward and backward passes in a forward/backpropagation algorithm. While adapting the parameters, the aggregated, for example summed, residues of all the training pairs are considered up to the current pair of for the current batch, to improve the objective function $F$.

The training system as shown in Fig. 4 can be considered for all learning schemes, in particular supervised schemes. Unsupervised learning schemes may also be envisaged herein in alternative embodiments. GPUs may be used to implement the training system TS. The fully trained machine learning module M may be stored in one or more memories MEM' or databases, and can be made available as pre-trained machine learning modules M. The trained model may be made available in a cloud service. Access can either be offered free of charge or their use can be granted via license-pay or pay-per-use scheme.

As an extension to the above, the training principles envisaged herein have been explained largely with reference to data pairs $(x,y)^i$ with, for each training instance $i$, $x$ the training input and $y$ its associated target, and thus for supervised learning, this is not limiting. In particular, unsupervised learning approaches based on clustering for example, are also envisaged herein in alternative embodiments.

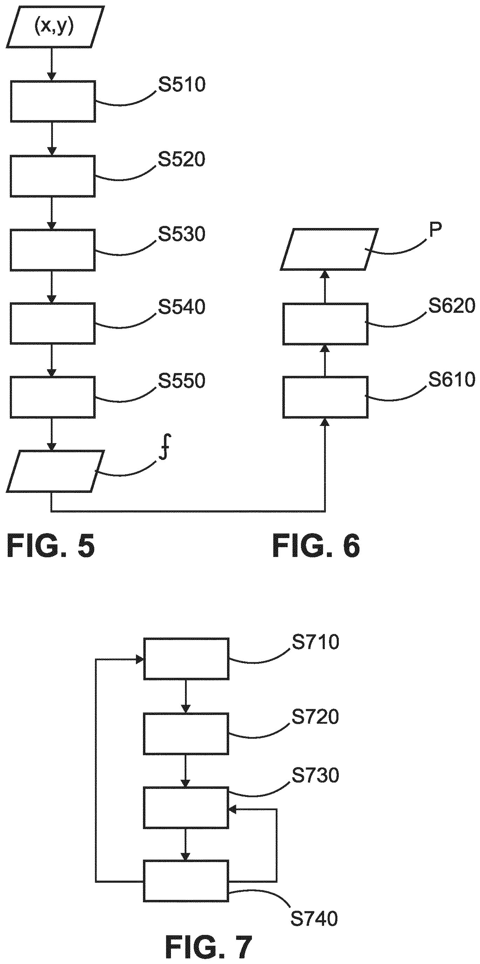

Reference is now made to flow charts in Figs. 5-7 which illustrate methods associable with the above-described facilitator system OGS. However, it should be understood that the below-described methods are not necessarily tied to the architecture shown above and may be understood as a teaching in their own right.

Broadly, Fig. 5 shows a flow chart of a computer-implemented method of facilitating generation of treatment plans. Objective function(s) are generated by this method. Fig. 6 is a flow chart for using the so generated objective functions $f = M$ in a plan optimization, whilst Fig. 7 shows steps of a method of training a machine model M.

Referring now first and in more detail to flow chart in Fig. 5, at step S510 training input data x is received from an existing training data corpus of previous RT plans. Selection of training input data for training may be facilitated by a user interface. The interface may allow effecting visual presentation of the plans from the corpus and selection of plans that are deemed by the user to represent plans that correspond in various degrees to a certain clinical goal or objective. The training data items include in particular different training plans or parts thereof for the same or different patients. Particularly, the training input data may include respective dose distribution maps (in 3D or 2D) of previous (selected) plans, optionally associated with other, in particular contextual data from the plan, such as patient medical data, planning imagery, indications of PTV, OAR(s), etc.

At step S520 the so selected input training data items $x$ are ranked preferably according to user response facilitated by a user interface. For example, a number (score) $y$ is awarded to each of the received training data items. The numbers are modulated to reflect the users view on the different levels/degree of correspondence or comportance of the data items with the goal/objective. For best results, it is recommended to choose a balanced selection of plans to reflect a good part/full range of the score scale. Preferably, user should not only restrict their selection to, in their view, good plans, but also include plans that are "negative" examples that do not comport well, again in their subjective view, with the medical goal. To aid the user with achieving such balanced selection, the input interface UI-T may be configured to support a sampler monitoring functionality. The sampler monitoring functionality indicates, preferably in a displayed and updatable graphic widget, the proportion of selected training samples over the pre-defined scoring range. This allows the user to adjust their training input data sampling from the corpus so that the proportions are approximately even over the range. The ranking operation may be explicit by the user specifying scores on the pre-defined scale, or may be implicit by mapping a sequence/ordered set, in which the selected plan are arranged by the user, to a predefined interval. Thus, in the latter case, the scoring is awarded automatically based on the user-prescribed order of the selected historical plans.

At step S530 the relationship between the awarded ranks and the historic plans are learned. Preferably, machine learning is used by adapting parameters of the model using a learning algorithm.

At an optional step S540, once the model has been learned (at the conclusion of step S530), this model is turned into an objective function, for example, into a look-up table or by using other functional approximation representations. However, this step is optional as it is the trained model itself that is used as an objective function for radiation treatment planning, and this is preferred herein. In other words, the learning step S530 generates the (one or more) objective function $f$ as the learned model.

At step S550 the objective function is stored in a memory or made otherwise available for use by or in a planning algorithm.

The storing of the objective function at step S550 may further include assigning identifiers or descriptors, preferably in natural language or other strings, to represent the respective medical goal that the user had in mind when awarding the ranking scores.

Turning now to Fig. 6, at step S610 the previously generated objective function $f$ (for example as per the method of Fig. 5) is received, preferably based on user selection as not all generated objective functions $f$ may be needed for each plan. Such selection may be facilitated by receiving corresponding requests by the user through a user interface UI-S.

At step S620 the learned (one or more) objective function $f$ is used in an optimization procedure. For example, the objective function $f$ may be incorporated into the plan optimization procedure to compute a new treatment plan P. At the conclusion of the panning optimization, the new plan may be output and may then be used for treating the patient in a radiation treatment session.

The plan P may be used to control the delivery device TD during the treatment. The objective function is preferably differentiable and can be used in gradient based optimization schemes.

The optimization step S620 may include in particular adapting an initial plan in one or more iterations to arrive at the final plan P. In the iterations, intermediate plans P' are generated. These intermediate plans are monitored based on the objective function $f$.

Specifically, the intermediate treatment plan is supplied to the objective function to compute the associated ranking, which is now interpreted as a figure of merit. The treatment plan parameters are adjusted so as to improve the response of the objective functions during the iterations of the plan optimization.

Objective functions as constructed in Fig. 5 are preferably differentiable and may be used in gradient based or other techniques where differentiability is a requirement. The manner in which the current set of plan parameters are adjusted may depend not only on the ranking score for the current parameters, but on the gradient. Based on current score and on the gradient, current parameters of plan are updated, and step S620 is repeated until convergence is detected or until a stopping condition is fulfilled, at which point the plan P is deemed final and can be released for use in treatment delivery. The parameters of the plan may include in particular the deliverable dose map.

The flow chart in Fig. 7 provides further details on the learning step S530.

Broadly, the training method includes receiving training data. Based on the training data, parameters of model M are adapted. The adaption may be iterative, and controlled by cost function $F$. Cost function F is configured to measure how well the model performs.

In more detail, and in some embodiments, at step S710 training data is received in the form of pairs $(x_k, y_k)$. Each pair includes the training input $x_k$ and the associated target $y_k$. $x_k$, as defined above.

At step S720, the training input $x_k$ or plural such training inputs of a batch is applied to an initialized machine learning model M to produce a training output.

A deviation, or residue, of the training output $M^\theta(x_k)$ from the associated target $y_k$ is quantified by a cost function $F$ at step S730. One or more parameters of the model are adapted at step S740 in one or more iterations in an inner loop to improve the cost function. For instance, the model parameters are adapted to decrease residues as measured by the cost function. The parameters include in particular weights W of the convolutional operators, in case a convolutional NN model M is used.

The training method then returns in an outer loop to step S710 where the next pair of training data or a next batch of such training data pairs is fed in. In step S720, the parameters of the model are adapted so that the aggregated residues of all pairs considered are decreased, in particular minimized. The cost function quantifies the aggregated residues. Forward/backward propagation or similar gradient-based techniques may be used in the inner loop.

More generally, the parameters of the model NN are adjusted to improve objective function $F$ which is either a cost function or a utility function. In embodiments, the cost function is configured to the measure the aggregated residues. In embodiments, the aggregation of residues is implemented by summation over all or some residues for all pairs considered. The method may be implemented on one or more general-purpose processing units TS, preferably having processors capable for parallel processing to speed up the training.

Components of system OGS may be implemented as one or more software modules, run on one or more general-purpose processing units PU such as a workstation associated with the treatment device TD, or on a server computer associated with a group of treatment devices TD or imagers IA.

Alternatively, some or all components of system OGS may be arranged in hardware such as a suitably programmed microcontroller or microprocessor, such as FPGA (field-programmable-gate-array), GPU, or as a hardwired IC chip, an application specific integrated circuitry (ASIC), integrated into the imaging system OGS. In a further embodiment still, system OGS may be implemented in both, partly in software and partly in hardware.

Different components of system OGS may be implemented on a single data processing unit PU. Alternatively, some or more components are implemented on plural processing units PU, possibly remotely arranged in a distributed architecture and communicatively couplable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

**Claims**

1. System (OGS) configured for generating an objective function for radiation treatment, RT, planning.

2. System of claim 1, wherein the system is based on a machine learning model (M), and the system comprises a training system (TS) configured to train the machine learning model based on training data.

3. System of claim 2, wherein the objective function is, or comprises, the trained machine learning model.

4. System of any one of the previous claims, comprising a user input interface (UI-T), configured to allow a user for providing the training data, the training data including i) plurality of previous at least partial radiation treatment plans and ii) a user defined ranking of the plurality of previous at least partial radiation treatment plans.

5. System of claim 4, wherein the user inface (UI-T) includes a graphical user interface.

6. System of claim 5, wherein the, or a, user interface (UI-O) is configured to allow the user to store the representation of the generated objective function in a data storage (MEM), preferably in association with a user selectable identifier that identifies an objective criterion.

7. System of claim 5, wherein the, or a, user interface (UI-S) is configured to allow the user to select the objective criterion, wherein, upon such selection, the objective function is included as an objective into an RT planner system (PS), the RT planner system (PS) to run an optimizing procedure driven at least by the objective function to compute a new treatment plan.

8. System of claim 7, wherein the optimizing procedure is of the inverse planning type.

9. System of any one of previous claims, wherein the machine learning model is a regression type model or is of the generative type.

10. System (PS) for computer-assisted radiation treatment planning, comprising:

    input interface (IN) for receiving a selection of an objective function generated by a system of any one of the previous claims; and
    an RT planning module (PL) configured to run an optimizing procedure driven at least by the selected objective function to compute a new treatment plan.

11. An RT arrangement (AR), comprising the system of claim 10, and a radiation delivery apparatus (TD) controllable by the new treatment plan.

12. A computer implemented method for supporting RT planning, comprising generating (S530) an objective function for RT planning.

13. Method of claim 12, further comprising providing (S550) the objective function to a RT planning module for computing an RT plan based on the objective function.

14. A computer program element, which, when being executed by at least one processing unit, is adapted to cause the at least one processing unit (PU) to perform the method as per claim 12 or 13.

15. At least one computer readable medium having stored thereon any one of more of i) the program element of claim 14, ii) the machine learning model as per claim 2.

**FIG. 1**

**FIG. 2**

**FIG. 3**

**FIG. 4**

**FIG. 5**

**FIG. 6**

**FIG. 7**

Europäisches
Patentamt

European
Patent Office

Office européen
des brevets

# EUROPEAN SEARCH REPORT

Application Number

EP 21 20 8387

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/206533 A1 (LAAKSONEN HANNU MIKAEL [FI] ET AL) 2 July 2020 (2020-07-02) | 1-3,5-15 | INV.<br>A61N5/10 |
| Y | * figures 1-3,6 *<br>* paragraph [0001] *<br>* paragraph [0004] *<br>* paragraph [0005] *<br>* paragraph [0007] *<br>* paragraph [0009] *<br>* paragraph [0013] *<br>* paragraph [0023] *<br>* paragraph [0029] – paragraph [0031] *<br>* paragraph [0048] *<br>* paragraph [0051] *<br>* paragraph [0052] *<br>* paragraph [0054] *<br>* paragraph [0060] *<br>* paragraph [0061] *<br>* paragraph [0066] – paragraph [0071] *<br>* paragraph [0075] *<br>* paragraph [0076]; claims 1,5,14,19,20 *<br>* paragraph [0082] * | 4 | G06N3/08<br>G06N20/00<br>G16H20/30<br>G16H20/40<br>G06N5/00 |
| X | US 2021/069527 A1 (PELTOLA JARKKO [FI] ET AL) 11 March 2021 (2021-03-11) | 1-3,5-15 | TECHNICAL FIELDS SEARCHED (IPC)<br>A61N |
| Y | * figures 1-6 *<br>* paragraph [0001] *<br>* paragraph [0069] *<br>* paragraph [0070] *<br>* paragraph [0080] *<br>* paragraph [0083] *<br>* paragraph [0087] *<br>* paragraph [0090] – paragraph [0091] *<br>* paragraph [0247] – paragraph [0248] *<br>* paragraph [0092] * | 4 | G06N<br>G16H |

-/--

The present search report has been drawn up for all claims

| Place of search | Date of completion of the search | Examiner |
|---|---|---|
| Munich | 7 April 2022 | Rodríguez Cosío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 21 20 8387

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 352 851 A1 (VARIAN MEDICAL SYSTEMS INT AG [CH]) 1 August 2018 (2018-08-01) | 1,10-14 | |
| Y | * paragraph [0001] * <br> * paragraph [0017] * <br> * paragraph [0020] * <br> * paragraph [0024] * <br> * paragraph [0027] * <br> ----- | 2-9,15 | |
| X | US 2020/009401 A1 (KUUSELA ESA [FI] ET AL) 9 January 2020 (2020-01-09) | 1,10-14 | |
| Y | * paragraph [0045] * <br> * paragraph [0076] * <br> * paragraph [0100] * <br> * paragraph [0103] * <br> * paragraph [0008] * <br> * paragraph [0107] * <br> * paragraph [0108] * <br> ----- | 2-9,15 | |
| Y | US 2021/027878 A1 (HE ZE [US] ET AL) 28 January 2021 (2021-01-28) <br> * paragraph [0001] * <br> * paragraph [0024] * <br> * paragraph [0039] * <br> * paragraph [0041] * <br> * paragraph [0042] * <br> * paragraph [0051]; claim 12 * <br> * paragraph [0060] * <br> ----- | 4,9 | TECHNICAL FIELDS SEARCHED (IPC) |
| Y | US 2012/280135 A1 (BAL MATTHIEU [NL]) 8 November 2012 (2012-11-08) <br> * paragraph [0001] * <br> * paragraph [0022] * <br> * paragraph [0029] * <br> ----- <br> -/-- | 4 | |

The present search report has been drawn up for all claims

| Place of search | Date of completion of the search | Examiner |
|---|---|---|
| Munich | 7 April 2022 | Rodríguez Cosío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt

European Patent Office

Office européen des brevets

# EUROPEAN SEARCH REPORT

Application Number

**EP 21 20 8387**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2021/145519 A1 (MOSNIER THOMAS [FR] ET AL) 20 May 2021 (2021-05-20)<br>* paragraph [0002]; claim 10 *<br>* paragraph [0179] *<br>* paragraph [0372] *<br>----- | 4,9 | |
| Y | US 2003/130573 A1 (YU YAN [US] ET AL) 10 July 2003 (2003-07-10)<br>* paragraph [0002] *<br>* paragraph [0059] *<br>----- | 4 | |
| Y | US 2021/220673 A1 (KAMERLING CORNELIS [DE] ET AL) 22 July 2021 (2021-07-22)<br>* paragraph [0001] *<br>* paragraph [0073] *<br>----- | 4 | |
| Y | US 2019/030370 A1 (HIBBARD LYNDON S [US]) 31 January 2019 (2019-01-31)<br>* paragraph [0009] *<br>* paragraph [0010] *<br>* paragraph [0011] *<br>----- | 9 | |
| Y | US 2021/142162 A1 (JEON SEOK HOON [KR] ET AL) 13 May 2021 (2021-05-13)<br>* paragraph [0001] *<br>* paragraph [0013] *<br>* paragraph [0034] – paragraph [0035] *<br>* paragraph [0036] *<br>* paragraph [0061] *<br>* paragraph [0063] *<br>* paragraph [0066] *<br>-----<br>-/-- | 9 | TECHNICAL FIELDS SEARCHED (IPC) |

The present search report has been drawn up for all claims

| Place of search | Date of completion of the search | Examiner |
|---|---|---|
| Munich | 7 April 2022 | Rodríguez Cosío, J |

EPO FORM 1503 03.82 (P04C01)

EP 4 180 088 A1

Europäisches Patentamt
European Patent Office
Office européen des brevets

# EUROPEAN SEARCH REPORT

Application Number

EP 21 20 8387

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2020/398079 A1 (ADELSHEIM CHARLES [US] ET AL) 24 December 2020 (2020-12-24)<br>* paragraph [0003] *<br>* paragraph [0026] *<br>* paragraph [0028] *<br>* paragraph [0091] – paragraph [0092] *<br>* paragraph [0093] *<br>----- | 4,9 | |
| A | US 2020/289847 A1 (SJÖLUND JENS OLOF [SE] ET AL) 17 September 2020 (2020-09-17)<br>* paragraph [0010] – paragraph [0011] *<br>----- | 1-15 | |
| A | US 2021/268313 A1 (GUNNARSSON NIKLAS [SE] ET AL) 2 September 2021 (2021-09-02)<br>* paragraph [0073] *<br>----- | 1-15 | |
| A | US 2018/322942 A1 (STANKIEWICZ BRIAN J [US] ET AL) 8 November 2018 (2018-11-08)<br>* paragraph [0087] *<br>----- | 1-15 | |
| A | US 2021/027109 A1 (ITO SHINJI [JP] ET AL) 28 January 2021 (2021-01-28)<br>* paragraph [0017] *<br>* paragraph [0004] *<br>----- | 1-15 | **TECHNICAL FIELDS SEARCHED (IPC)** |

The present search report has been drawn up for all claims

| Place of search | Date of completion of the search | Examiner |
|---|---|---|
| Munich | 7 April 2022 | Rodríguez Cosío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 20 8387

This annex lists the patent family members relating to the patent documents cited in the above-mentioned European search report.
The members are as contained in the European Patent Office EDP file on
The European Patent Office is in no way liable for these particulars which are merely given for the purpose of information.

07-04-2022

| Patent document cited in search report | Publication date | Patent family member(s) | Publication date |
|---|---|---|---|
| US 2020206533 A1 | 02-07-2020 | NONE | |
| US 2021069527 A1 | 11-03-2021 | US 2021069527 A1 | 11-03-2021 |
| | | WO 2021048143 A1 | 18-03-2021 |
| EP 3352851 A1 | 01-08-2018 | EP 3352851 A1 | 01-08-2018 |
| | | HK 1259345 A1 | 29-11-2019 |
| | | US 2017091387 A1 | 30-03-2017 |
| | | US 2020043580 A1 | 06-02-2020 |
| | | US 2022036983 A1 | 03-02-2022 |
| | | WO 2017049415 A1 | 30-03-2017 |
| US 2020009401 A1 | 09-01-2020 | CN 110114117 A | 09-08-2019 |
| | | EP 3562549 A1 | 06-11-2019 |
| | | EP 3741431 A1 | 25-11-2020 |
| | | EP 3744395 A1 | 02-12-2020 |
| | | US 2018185669 A1 | 05-07-2018 |
| | | US 2020009401 A1 | 09-01-2020 |
| | | WO 2018122251 A1 | 05-07-2018 |
| US 2021027878 A1 | 28-01-2021 | CN 111989749 A | 24-11-2020 |
| | | EP 3769317 A1 | 27-01-2021 |
| | | JP 2021518196 A | 02-08-2021 |
| | | US 2021027878 A1 | 28-01-2021 |
| | | WO 2019179857 A1 | 26-09-2019 |
| US 2012280135 A1 | 08-11-2012 | CN 102656607 A | 05-09-2012 |
| | | EP 2513863 A1 | 24-10-2012 |
| | | JP 5767242 B2 | 19-08-2015 |
| | | JP 2013514115 A | 25-04-2013 |
| | | RU 2012130028 A | 27-01-2014 |
| | | US 2012280135 A1 | 08-11-2012 |
| | | WO 2011073820 A1 | 23-06-2011 |
| US 2021145519 A1 | 20-05-2021 | NONE | |
| US 2003130573 A1 | 10-07-2003 | AU 2002322662 A1 | 17-02-2003 |
| | | US 2003130573 A1 | 10-07-2003 |
| | | WO 03011390 A2 | 13-02-2003 |
| US 2021220673 A1 | 22-07-2021 | EP 3497702 A1 | 19-06-2019 |
| | | US 2021220673 A1 | 22-07-2021 |
| | | WO 2019081042 A1 | 02-05-2019 |
| US 2019030370 A1 | 31-01-2019 | AU 2018307739 A1 | 05-03-2020 |
| | | CN 110944717 A | 31-03-2020 |

EPO FORM P0459

## ANNEX TO THE EUROPEAN SEARCH REPORT
## ON EUROPEAN PATENT APPLICATION NO.

EP 21 20 8387

This annex lists the patent family members relating to the patent documents cited in the above-mentioned European search report.
The members are as contained in the European Patent Office EDP file on
The European Patent Office is in no way liable for these particulars which are merely given for the purpose of information.

07-04-2022

| Patent document cited in search report | Publication date | Patent family member(s) | Publication date |
|---|---|---|---|
| | | EP 3658231 A1 | 03-06-2020 |
| | | JP 6934559 B2 | 15-09-2021 |
| | | JP 2020528791 A | 01-10-2020 |
| | | JP 2021183173 A | 02-12-2021 |
| | | US 2019030370 A1 | 31-01-2019 |
| | | WO 2019023142 A1 | 31-01-2019 |
| US 2021142162 A1 | 13-05-2021 | KR 20210057430 A | 21-05-2021 |
| | | US 2021142162 A1 | 13-05-2021 |
| US 2020398079 A1 | 24-12-2020 | CN 113795303 A | 14-12-2021 |
| | | EP 3986547 A1 | 27-04-2022 |
| | | US 2020398079 A1 | 24-12-2020 |
| | | WO 2020257216 A1 | 24-12-2020 |
| US 2020289847 A1 | 17-09-2020 | NONE | |
| US 2021268313 A1 | 02-09-2021 | NONE | |
| US 2018322942 A1 | 08-11-2018 | AU 2016349861 A1 | 24-05-2018 |
| | | CA 3004259 A1 | 11-05-2017 |
| | | EP 3371771 A1 | 12-09-2018 |
| | | US 2018322942 A1 | 08-11-2018 |
| | | WO 2017079012 A1 | 11-05-2017 |
| US 2021027109 A1 | 28-01-2021 | JP WO2019187289 A1 | 11-03-2021 |
| | | US 2021027109 A1 | 28-01-2021 |
| | | WO 2019187289 A1 | 03-10-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL.** Machine Learning. McGraw-Hill, 1997, 2.1.1-6.1.2.1
- **D CRAFT.** *Multi-criteria optimization methods in radiation therapy planning,* May 2018
- **IAN GOODFELLOW et al.** *Generative Adversarial Networks,* 2014